(19) Europäisches Patentamt
European Patent Office
Office européen des brevets

(11) Publication number: 0 019 946 B1

(12) # EUROPEAN PATENT SPECIFICATION

(45) Date of publication of patent specification: 25.05.83

(51) Int. Cl.$^3$: C 07 D 417/06, A 01 N 43/86 //C07D279/06, C07D333/24, C07D307/54

(21) Application number: 80200351.7

(22) Date of filing: 17.04.80

(54) **Dihydrothiazine derivatives, their preparation, their inclusion in anthelmintic and pesticidal compositions and their use as anthelmintics and pesticides.**

(30) Priority: 23.05.79 GB 7917981

(43) Date of publication of application: 10.12.80 Bulletin 80/25

(45) Publication of the grant of the patent: 25.05.83 Bulletin 83/21

(84) Designated Contracting States: BE CH DE FR GB IT LI NL

(56) References cited:
US - A - 3 637 707
US - A - 4 045 434

(73) Proprietor: INTERNATIONAL MINERALS & CHEMICAL CORPORATION
2315 Sanders Road
Northbrook, Illinois (US)

(72) Inventor: Anderson, Martin
92 Clare Road
Whitstable, Kent C7 52GB (GB)
Inventor: Woodall, Roger Ernest
Kestrels Chequers Hill Doddington
Sittingbourne, Kent, ME 90BN (GB)

(74) Representative: Allard, Susan Joyce et al,
BOULT, WADE & TENNANT 27 Furnival Street
London EC4A 1PQ (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 019 946 B1

Courier Press, Leamington Spa, England

Dihydrothiazine derivatives, their preparation, their inclusion in anthelmintic and pesticidal compositions and their use as anthelmintics and pesticides

This invention relates to the dihydrothiazine derivatives, their preparation, their inclusion in anthelmintic and pesticidal compositions and their use as anthelmintics and pesticides.

US—A—3637707 discloses certain 2-(substituted)-2-thiazolines and 2-(substituted)-5,6-dihydro-4H-1,3-thiazines for the prevention of rice blast on rice plants. In these compounds a group Y which is $—CH{=}CH—$, $—C(CH_3){=}CH—$, $—CH{=}C(CH_3)—$, $—C(OH){=}CH—$ or $—(CH_2)_n—$ is attached to the thiazoline or thiazine ring.

US—A—4045434 discloses certain esters of nitro (tetrahydro-2H-1,3-thiazin-2-ylidene)acetic acids which possess insecticidal activity. In these compounds a nitro group is always attached directly to the $\alpha$-carbon atom of the heterocyclic thiazine group.

According to the invention there is provided a dihydrothiazine derivative of formula I

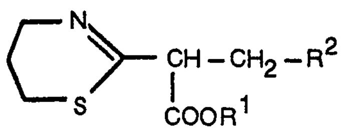

(I)

or a tautomer thereof, wherein $R^1$ is a $C_{1-10}$ alkyl, $C_{2-10}$ alkynyl, $C_{3-10}$ cycloalkyl or $C_{4-11}$ cycloalkylalkyl group optionally substituted by halogen, or is a phenyl or benzyl group optionally substituted by halogen, amino, or nitro and $R^2$ is a thienyl or furyl group optionally substituted by alkyl containing from 1 to 4 carbon atoms, or a non-toxic acid-addition salt thereof, provided that when $R^1$ is an alkenyl or alkynyl group the double or triple bond, respectively, is not in the $\alpha$-position to the oxygen in the group $—COOR^1$.

It can readily be seen that the compounds of the invention differ from the compounds disclosed in the two United States Patent Specifications referred to above.

The dihydrothiazine derivatives of formula I usually exist as equilibrium mixtures with their tautomers of formula I(a)

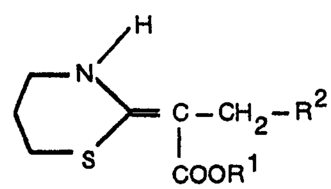

(Ia)

It will be appreciated that usually one or other of the tautomeric forms will predominate, possibly to the virtual exclusion of the other forms, depending upon the nature of the $R^1$ and $R^2$ groups and the environment of the compounds (e.g. whether the compounds are isolated in pure forms, or as acid-addition salts, or are in solution). It will also be understood that the present invention extends to the individual tautomeric forms and to mixtures thereof.

The dihydrothiazine derivatives of formula I may form acid-addition salts, and the invention also extends to non-toxic acid-addition salts of the dihydrothiazine derivatives of formula I, e.g. to salts such as hydrochloride and tartrates.

Preferred dihydrothiazine derivatives of formula I are those having one or more of the following features:

(i) $R^1$ represents $C_{1-10}$ alkyl, $C_{4-11}$ cycloalkylalkyl or $C_{3-10}$ cycloalkyl,

(ii) $R^1$ represents $C_{6-10}$ cycloalkyl or $C_{4-7}$ cycloalkylalkyl,

(iii) $R^1$ represents $C_{1-4}$ alkyl or cyclopropylmethyl,

(iv) $R^2$ represents an optionally substituted thienyl or furyl group.

(v) $R^2$ represents a 2-thienyl or 2-furyl group optionally substitued by one or more $C_{1-4}$ alkyl groups, and

(vi) $R^2$ represents a 2-thienyl group optionally substituted in the 3-position by a methyl group.

Preferably dihydrothiazine derivatives of formula I have one of features (i) to (iii) and also one of features (iv) to (vi). The most preferred dihydrothiazine derivative of the invention are those having features (iii) and (vi).

The invention further provides a process for preparing a dihydrothiazine derivative of formula I which comprises reacting a compound of formula II

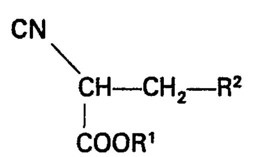

(II)

where $R^1$ and $R^2$ are as defined above, with 3-aminopropanethiol to give a compound of formula I, optionally followed by exchanging the group $R^1$ for another group $R^1$ as defined above.

The reaction of the compound of formula II with 3-aminopropanethiol is advantageously carried out at elevated temperature in an inert solvent under an inert gas, e.g. nitrogen. For example, the reaction may be carried out in methylene chloride, at reflux temperature. If the 3-aminopropanethiol is initially in the form of an acid-addition salt, such as the hydrochloride, the reaction is desirably carried out in the presence of an organic base such as triethylamine.

The optional ester exchange reaction whereby one $R^1$ group in the compound of formula I is substituted by a different $R^1$ group may in general be effected by dissolving potassium metal in the appropriate hydroxy compound $R^1$—OH, advantageously in the presence of an inert solvent such as toluene, and adding to the resulting mixture the compound of formula I (e.g. wherein $R^1$ is methyl) and an appropriate molecular sieve (e.g. size 4A when the $R^1$ group to be removed is methyl). The resulting suspension is heated, conveniently at reflux temperature, and the moelcular sieve is subsequently removed by filtration.

The intermediates of formula II may be prepared by reduction of compounds of formula III

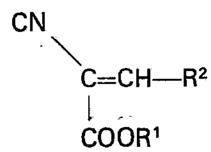

(III)

where $R^1$ and $R^2$ are as defined above. The reduction is conveniently effected using sodium borohydride similarly to the method of Kompis and Schönholzer, Helv. Chim. Acta, 1977, *60*, 618. However, in suitable cases, e.g. when $R^2$ is thienyl or alkylthienyl catalytic hydrogenation, using, for example, palladium on charcoal as catalyst, may be employed.

The compounds of formula III may be prepared by Knoevanagel condensation of the appropriate aromatic aldehyde of formula IV

$$R^2\text{—CHO} \qquad \text{(IV)}$$

where $R^2$ is as defined above, with the appropriate cyanoacetic ester of formula V

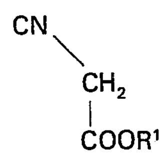

(V)

where $R^1$ is as defined above, under conditions similar to those described by Popp. J. Org. Chem., 1960, *25*, 646.

Dihydrothiazine derivatives of formula I have utility as anthelmintics and as pesticides. They have low mammalian toxicity.

Accordingly the invention also provides an anthelmintic or pesticidal composition comprising, as active ingredient, a dihydrothiazine derivative of formula I in association with a non-toxic carrier therefor. The invention further provides a method of preparing such a composition which comprises bringing a dihydrothiazine derivative of formula I into association with a non-toxic carrier therefor. Also provided in accordance with the invention is the use of dihydrothiazine derivative of formula I as an anthelmintic or as a pesticide.

The compounds of the invention are effective in combatting parasitic worms, particularly those which are present in the alimentary tract of an animal when the dihydrothiazine derivative is orally administered to the animal. The compounds are particularly effective in combatting parasitic worms in ruminants, e.g. sheep, pigs, cattle and goats.

Oral administration of the dihydrothiazine derivatives of formula I may be by any convenient means, e.g. as a drench, by intubation, in the animal's food and water, in a food supplement or in specific formulations such as solvents, suspensions, dispersions, emulsions, tablets, boluses, powders, granules, capsules, syrups and elixirs. Suitable non-toxic carriers are the conventional inert pharmaceutically-acceptable carriers such as water, edible oils, gelatin, lactose, starch, magnesium stearate, talc or vegetable gum.

The dosage of the dimhydrothiazine derivative needed to combat parasitic worms will depend on the particular derivative used and the animal being treated. However in general the derivative will be administered in a dosage of from 5 to 200 milligrams per kilogram of the animal's body weight, typically from 10 to 30 mg/kg for sheep, pigs, goats and cattle. The derivative can be administered in a single dose or in a series of doses in a day, or series of days. For any particular animal, a specified dosage regimen should be adjusted according to the individual need, the particular derivative used and

the professional judgement of the person administering or supervising administration of the derivative.

The invention additionally provides a method of combatting pests, such as insect pests, at a locus which comprises applying to that locus a pesticidally-effective amount of dihydrothiazine derivative of formula I.

A carrier in a pesticidal composition of the invention may be a solid or a liquid, including a material which is normally gaseous but which has been compressed to form a liquid, inorganic or organic, and of synthetic or natural origin. The active ingredient is suitably formulated with at least one carrier to facilitate its application to the locus, for example, plants, seeds or soil, to be treated, or to facilitate storage, transport or handling.

Preferably a pesticidal composition of the invention contains at least two carriers, at least one of which is a surface-active agent. The surface-active agent may be an emulsifier, a dispersing agent or a wetting agent; it may be non-ionic or ionic. Pesticidal compositions are generally formulated and transported in a concentrated form which is subsequently diluted by the farmer or other user before application. A surface-active agent facilitates this process of dilution.

Any of the carriers commonly used in the formulation of pesticides may be used in pesticidal compositions of the invention, and suitable examples of these are to be found, for example, in GB—A—1,232,930.

Pesticidal compositions of the invention may for example be formulated as a wettable powder, microcapsules, a dust, granules, a solution, an emulsifiable concentrate, an emulsion, a suspension concentrate or an aerosol. The pesticidal composition may have controlled release properties, or may be suitable for use as a bait.

Wettable powders usually contain 25, 50 or 75% w of active ingredient and may contain, in addition to inert solid material, 3—10% w of a dispersing agent and, where necessary, 0—10% w of a stabiliser, a penetrant and/or a sticker. A dust is usually formulated as a dust concentrate having a composition similar to that of a wettable powder but without a dispersant, and is diluted in the field with further solid carrier to give a composition usually containing ½—10% w of active ingredient.

Granules usually have a size in the range of from 10 to 100 BS mesh (1.676—0.152 mm) and may be manufactured by agglomeration or impregnation techniques. Generally, granules will contain ½—25% w active ingredient and 0—10% w of additives, for example a stabiliser, slow release modifier and/or a binding agent.

Emulsifiable concentrates usually contain, in addition to a solvent, and, when necessary, cosolvent, 10—50% w/v active ingredient, 2—20% w/v emulsifier and 0—20% of other additives, for example a stabiliser, a penetrant and/or a corrosion inhibitor. A suspension concentrate is a stable, non-sedimenting, flowable product and usually contains 10—75% w active ingredient, 0.5—15% w of dispersing agent, 0.1—10% w of suspending agent, for example protective colloid and/or a thixotropic agent, and 0—10% w of other additives including, for example, a defoamer, a corrosion inhibitor, a stabiliser, a penetrant and/or a sticker, and as dispersant, water or an organic liquid in which the active ingredient is substantially insoluble; certain organic additives and/or inorganic salts may be dissolved in the dispersant to assist in preventing sedimentation or as anti-freeze for water.

The aqueous dispersions and emulsions formed by diluting a wettable powder or an emulsifiable concentrate of the invention with water, also lie within the scope of the present invention. Such dispersions and emulsions may be of the water-in-oil or of the oil-in-water type, and may have a thick "mayonnaise"-like consistency.

A pesticidal composition of the invention may also contain other ingredients, for example, one or more other compounds possessing pesticidal, herbicidal or fungicidal properties, or attractants, for example pheromones or food ingredients, for use in baits and trap formulations.

The invention will be better understood from the following illustrative Examples, of which Examples 9 to 15 and 18 relate to the preparation of compounds of the invention and Examples 1 to 8, 16 and 17 relate to intermediates.

Example 1

*Methyl 2-cyano-3-(2-thienyl)acrylate*

A solution of piperidine (1.0 ml) in dioxan (10.0 ml) was added carefully to a stirred solution of thiophene-2-carboxaldehyde (33.65 g, 0.3 moles) and methyl cyanoacetate (29.7 g, 0.3 moles) in dioxan (40 ml). On standing the reaction mixture overnight at room temperature a crystalline precipitate was obtained which was filtered off, washed with cold dioxan and dried *in vacuo* to give title product (yield 49.3 g, 85%) m.p. 104—106°C.

| Analysis | C | H | N |
|---|---|---|---|
| Calculated for $C_9H_7NO_2S$ | 55.9 | 3.7 | 7.2% |
| Found | 56.1 | 3.8 | 7.5% |

Examples 2 to 4

By methods similar to that of Example 1, there were prepared the following compounds:

*ethyl 2-cyano-3-(2-thienyl)acrylate* (yield 76%) mp 94—95°C

| Analysis | C | H | N |
|---|---|---|---|
| Calculated for $C_{10}H_9NO_2S$ | 57.9 | 4.4 | 6.8% |
| Found | 57.6 | 4.3 | 6.4% |

*t-butyl 2-cyano-3-(2-thienyl)acrylate* (yield 72%) mp 114—115°C

| Analysis | C | H | N |
|---|---|---|---|
| Calculated for $C_{12}H_{13}NO_2S$ | 61.2 | 5.6 | 5.9 |
| Found | 61.2 | 5.4 | 5.9 |

*methyl 2-cyano-3-(3-methylthien-2-yl)acrylate* (yield 92%) mp 135—136°C

| Analysis | C | H | N |
|---|---|---|---|
| Calculated for $C_{10}H_9NO_2S$ | 57.9 | 4.4 | 6.8% |
| Found | 57.9 | 4.3 | 6.6% |

Example 5

*Methyl 2-cyano-3-(2-thienyl)propionate*

Methyl 2-cyano-3-(2-thienyl)propionate (29 g, 0.15 moles) was dissolved in 300 ml of methanol/water (80:20 v/v). To the resulting solution was added about 3 drops of molar aqueous sodium hydroxide solution followed by sodium borohydride (1.8 g, 0.048 moles) and the suspension thus formed was stirred overnight at room temperature. The reaction mixture was then neutralised using molar aqueous acetic acid solution and volatile components were evaporated off under reduced pressure. The residue was suspended in water and extracted with toluene. The extract was dried over sodium sulphate and the toluene was evaporated off *in vacuo* to give a crude product which was purified by distillation to yield the title product (yield 10.6 g, 36%) bp 172°C/12 mm Hg (1599.8 Pa)

| Analysis | C | H | N |
|---|---|---|---|
| Calculated for $C_9H_9NO_2S$ | 55.4 | 4.6 | 7.2% |
| Found | 55.4 | 4.8 | 7.0 |

Examples 6 and 7

By methods similar to that of Example 5 there were also prepared:

*ethyl 2-cyano-3-(2-thienyl)propionate* (yield 52%) bp 112—114°C/0.25 mm Hg (33.33 Pa)

| Analysis | C | H | N |
|---|---|---|---|
| Calculated for $C_{10}H_{11}NO_2S$ | 57.4 | 5.3 | 6.7% |
| Found | 57.7 | 5.4 | 6.6 |

*t-butyl 2-cyano-3-(2-thienyl)propionate* (yield 81%) oil

Example 8

*Methyl 2-cyano-3-(3-methylthien-2-yl)propionate*

Methyl 2-cyano-3-(3-methylthien-2-yl)acrylate (75.0 g, 0.36 moles) was suspended in methanol (1.1) and hydrogenated in four batches in a Parr apparatus at 50°C and 4.23 atmospheres using 0.25 g of 10% palladium/charcoal catalyst per batch. Distillation of the residue remaining after removal of the catalyst and solvent gave the pure title compound as a pale yellow oil (70.4 g, 93%) bp 119°C/0.5 mm Hg (66.66 Pa).

| Analysis | C | H | N |
|---|---|---|---|
| Calculated for $C_{12}H_{15}NO_2S_2$ | 57.4 | 5.3 | 6.7% |
| Found | 57.5 | 5.5 | 6.7% |

Example 9

*Methyl 2-(5,6-dihydro-4H-1,3-thiazin-2-yl)-3-(2-thienyl)propionate*

A suspension of 3-aminopropanethiol hydrochloride (4.7 g, 0.037 moles) in methylene chloride (150 ml) containing methyl 2-cyano-3-(2-thienyl)propionate (6.5 g, 0.033 moles) and triethylamine (3.7 g, 0.037 moles) was stirred at reflux temperature under nitrogen for 17 hours. After filtering of the

reaction mixture the solvent was evaporated off under reduced pressure, leaving an oily residue which was suspended in water and extracted with ether. The ether extract was dried over sodium sulphate, the ether was evaporated off, and the residue was distilled under reduced pressure to give the title product as a pale yellow liquid (yield 3.4 g, 38%) bp 171°C/0.4 mm Hg (53.32 Pa).

| Analysis | C | H | N |
|---|---|---|---|
| Calculated for $C_{12}H_{15}NO_2S_2$ | 53.5 | 5.6 | 5.2% |
| Found | 53.8 | 5.6 | 4.8% |

Examples 10 to 12

The following compounds were prepared by methods similar to that used in Example 9:

*ethyl 2(5,6-dihydro-4H-1,3-thiazin-2-yl)-3-(2-thienyl)propionate* (yield 8%) bp 144—154°C/0.2 mm Hg (26.66 Pa).

| Analysis | C | H | N |
|---|---|---|---|
| Calculated for $C_{13}H_{17}NO_2S_2$ | 55.1 | 6.1 | 4.9% |
| Found | 55.5 | 6.1 | 5.2% |

*t-butyl 2(5,6-dihydro-4H-1,3-thiazin-2-yl)-3-(2-thienyl)propionate* (yield 38%) oil

| Analysis | C | H | N |
|---|---|---|---|
| Calculated for $C_{15}H_{21}NO_2S_2$ | 57.8 | 6.8 | 4.5% |
| Found | 58.1 | 6.8 | 4.5% |

*methyl 2(5,6-dihydro-4H-1,3-thiazin-2-yl)-3-(3-methylthien-2-yl) propionate* (yield 48%) bp 163—164°C/0.4 mm Hg (53.32 Pa).

| Analysis | C | H | N |
|---|---|---|---|
| Calculated for $C_{13}H_{17}NO_2S_2$ | 55.1 | 6.1 | 4.9% |
| Found | 55.8 | 6.4 | 5.2 |

Example 13

*n-propyl 2-(5,6-dihydro-4H-1,3-thiazin-2-yl)-3-(2-thienyl)propionate*

Potassium (0.1 g) was dissolved in *n*-propanol (6.0 g) was diluted with dry toluene (75 ml). To the resulting solution was added methyl 2(5,6-dihydro-4H-1,3-thiazin-2-yl)-3-(2-thienyl)propionate (5.4 g, 0.02 moles) (prepared as in Example 9) and size 4A molecular sieve (BDH reagent grade, which comprises 3.175 mm ($\frac{1}{8}$ inch) pellets of the sodium salt of zeolithe 30 g) which had previously been stored at 300°C. The resulting suspension was stirred vigorously at reflux temperature for 16 hours. After removal of the molecular sieve the solvent components were evaporated off under reduced pressure. The residue was subjected to chromatography on the silica gel column using diethyl ether as eluent to give the title product as an oil (yield 1.8 g, 30%).

| Analysis | C | H | N |
|---|---|---|---|
| Calculated for $C_{14}H_{19}NO_2S_2$ | 56.5 | 6.4 | 4.7% |
| Found | 56.8 | 6.6 | 4.8% |

Examples 14 and 15

The following compounds were prepared by methods similar to that of Example 14.

*n-butyl 2-(5,6-dihydro-4H-1,3-thiazin-2-yl)-3-(2-thienyl)propionate* (yield 32%) oil

| Analysis | C | H | N |
|---|---|---|---|
| Calculated for $C_{15}H_{21}NO_2S_2$ | 57.8 | 6.8 | 4.5% |
| Found | 58.1 | 6.8 | 4.7% |

*cyclopropylmethyl 2-(5,6-dihydro-4H-1,3-thiazin-2-yl)-3-(2-thienyl) propionate* (yield 48%) oil

| Analysis | C | H | N |
|---|---|---|---|
| Calculated for $C_{15}H_{19}NO_2S_2$ | 58.2 | 6.2 | 4.5% |
| Found | 58.1 | 6.2 | 4.5% |

Example 16

*Methyl 2-cyano-3-(2-furyl)acrylate*

A solution of piperidine (1.0 ml) in dioxan (10.0 ml) was added carefully to a stirred solution of

furfural (28.8 g, 0.3 moles) and methyl cyanoacetate (29.7 g, 0.3 moles) in dioxan (40 ml). After stirring at room temperature overnight, the reaction mixture was diluted with water (100 ml) and extracted with toluene. The extract was dried over sodium sulphate and the toluene was evaporated off *in vacuo* to give a crude product as a red solid. The crude product was crystallised from toluene/hexane to give the pure title product (yield 37.3 g, 70%) as red crystals, mp 90—91°C.

| Analysis | C | H | N |
|---|---|---|---|
| Calculated for $C_9H_7NO_3$ | 61.0 | 4.0 | 7.9% |
| Found | 61.1 | 4.2 | 8.2% |

Example 17

*Methyl 2-cyano-3-(2-furyl)propionate*

Methyl 2-cyano-3-(2-furyl)acrylate (26.4 g, 0.149 moles) was dissolved in 300 ml of methanol/water (80:20 v/v). About 3 drops of molar aqueous sodium hydroxide were added to the resulting solution, followed by sodium borohydride (1.8 g, 0.048 moles). The suspension thus formed was stirred at room temperature overnight and was then neutralised with molar aqueous acetic acid. Volatile components of the reaction mixture was evaporated off under reduced pressure and the residue was suspended in water and extracted with toluene. The extract was dried over sodium sulphate and the toluene was evaporated off *in vacuo* to give a crude product as red oil. The red oil was partially purified by chromatography on silica gel using toluene/methanol (9:1 v/v) as eluent and the partially purified material therefrom was distilled under reduced pressure to give the pure title product as a pale yellow liquid (yield 12.2 g, 46%) bp 82°C/0.2 mm Hg (26.66 Pa).

| Analysis | C | H | N |
|---|---|---|---|
| Calculated for $C_9H_9NO_3$ | 60.3 | 5.1 | 7.8% |
| Found | 59.0 | 5.0 | 8.1% |

Example 18

*Methyl 2-(5,6-dihydro-4H-1,3-thiazin-2-yl)-3-(2-furyl)propionate*

A suspension of 3-aminopropanethiol hydrochloride (7.33 g, 0.0575 moles) in dry methylene chloride (100 ml) containing methyl 2-cyano-3-(2-furyl)propionate (10.1 g, 0.0523 moles) and triethylamine (5.8 g, 0.0575 moles) was stirred at reflux temperature under nitrogen for 17 hours. After filtering of the reaction mixture, the solvent was evaporated off under reduced pressure, leaving an oily residue which was suspended in water and extracted with ether. The ether extract was dried over sodium sulphate, the ether was evaporated off, and the residue was distilled under reduced pressure to give the title product as a pale yellow liquid (yield 5.8 g, 44%) bp 158°C/0.4 mm Hg (53.32 Pa).

| Analysis | C | H | N |
|---|---|---|---|
| Calculated for $C_{12}H_{15}NSO_3$ | 56.9 | 6.8 | 5.5% |
| Found | 57.4 | 6.0 | 6.1% |

The parasiticidal and pesticidal activity of the compounds of the invention was determined by the following tests.

Test 1

The compounds were tested in an anthelmintic screen which has been found to have a high specificity for ruminant related nematodes and to detect known ruminant anthelmintics. The screen involves determining the efficacy of the compounds in treating rates infected with rat roundworm (*Nippostrongylus brasiliensis*).

White, male, Sprague-Dawley derived rats having a body weight of approximately 50 grams are exposed to *N. brasiliensis* infection by inoculation by subcutaneous injection in the cervical region with 1 millilitre of water containing about 200 *N. brasilensis* larvae. The rats are then held for 9 days for maturation of the infection.

Each test compound is weighed in 50 mg amounts into glass vials with screw caps. When prepared for testing, the test compound is triturated in a mortar and 6 cc corn oil added as a vehicle. If a test compound will not suspend well or go into solution in the corn oil when triturated in the mortar, mixture is subjected to ultrasonic oscillation and the associated heat which generally accomplishes the desired effect. Following the formulation of the test compound in the corn oil vehicle, the mixture is incorporated into 200 grams of ground rat chow. Using round-bottom bowls and a hand-held electric mixer, the test compound, corn oil, and feed are well mixed. The medicated feed is then transferred to labelled plastic bags for filling feeders.

On the ninth day following exposure to the parasitic infection, rats are randomly distributed to plastic holding boxes. Two rats are used as a treatment group for each of a series of different dose rates of each test compound. Five to ten non-medicated controls are also included. Special self-feeding feeders containing the medicated or non-medicated feeds are then added to each box along with an identification card. Following a 5-day medicated feed treatment period, the rats are transferred to

special observation cubicles for a 24-hour fast prior to necropsy. The rats are sacrificed using carbon dioxide ("dry ice"). The proximal 10 to 15 inches of the small intestine is removed, compressed under heavy glass plates, and the numbers of small red parasitic worms are counted by eye.

The test results are given in Table I.

TABLE I

| Test compound of Example | Concentration of test compound in feed (ppm) | Calculated total dose of test compound per rat (mg/kg) | Number of animals tested | Number of animals cleared of worms | % reduction in number of worms compared with control |
|---|---|---|---|---|---|
| 9 | 100 | 60 | 4 | 2 | 96 |
| | 30 | 18 | 4 | 1 | 60 |
| 10 | 300 | 180 | 2 | 2 | 100 |
| | 250 | 150 | 2 | 2 | 100 |
| | 100 | 60 | 2 | 2 | 100 |
| | 30 | 18 | 2 | 0 | 48 |
| 11 | 300 | 180 | 2 | 0 | 98 |
| | 250 | 150 | 2 | 1 | 79 |
| | 100 | 60 | 2 | 0 | 48 |
| 12 | 300 | 180 | 2 | 2 | 100 |
| | 100 | 60 | 2 | 2 | 100 |
| | 30 | 18 | 2 | 0 | 74 |
| 13 | 250 | 150 | 2 | 2 | 100 |
| | 100 | 60 | 2 | 2 | 100 |
| | 30 | 18 | 2 | 0 | 90 |
| 14 | 300 | 180 | 2 | 2 | 100 |
| | 250 | 150 | 2 | 2 | 100 |
| | 100 | 60 | 2 | 2 | 100 |
| | 30 | 18 | 2 | 0 | 43 |
| 15 | 300 | 180 | 2 | 2 | 100 |
| | 200 | 120 | 2 | 2 | 100 |
| | 100 | 60 | 2 | 2 | 100 |
| | 30 | 18 | 2 | 0 | 79 |

Test 2

The compound of Example 12, its tartrate salt, and the tartrate salt of the compound of Example 9 were tested for anthelmintic activity in sheep.

Twenty three sheep having body weights in the range 25 to 35 kg and substantial fecal parasite egg counts (>1000) were randomly assigned to one of three treatment groups of five sheep each, or a control group of eight sheep. Five days prior to administration of test compound, each group was moved to a respective holding pen where food (Happy Holme Farms, MD Lamb Fattener # 3) and water were provided on a free-choice basis.

Based upon pretreatment body weights, individual dosages of the compound of Examples 12 at 15 mg/kg, the tartrate salt of the compound of Example 12 at 15 mg/kg, and the tartrate salt of the compound of Example 9 at 25 mg/kg, were prepared. The compound were formulated by adding one drop of Tween 80 emulsifier ("Tween" is a trade mark) while mixing the compound with a small volume of water. Where ready mixing did not occur, ultrasonic oscillation was used to disperse the compound. Additional water was added to bring total volume of each dosage to 35 ml. The dosage were administered as a single oral drench, control animals being given plain water.

On the fifth day after treatment all the sheep were taken off food following the morning feed. Thirty-six hours later, necropsies were performed for each animal. The abomasum, small intestine and cecum/colon of each sheep were removed and the contents washed over a 0.25 mm screen. The abomasum and small intestine of each sheep were subjected to an artificial digestion overnight, and

once again washed over a 0.25 mm screen. All washings were placed in a 10% formalin solution, and following conventional procedures, all of the parasites were collected, identified, counted and tabulated, and percentage reductions in infestations of the treated animals were calculated. Results are given in Table II.

TABLE II

| Parasite species | Percent Reduction | | |
|---|---|---|---|
| | Compound of Example 12 (15 mg/kg) | Tartrate salt of compound of Example 12 (15 mg/kg) | Tartrate salt of compound of Example 9 (25 mg/kg) |
| *Haemonchus* | 84.9 | 47.6 | 78.2 |
| *Ostertagia* | 79.0 | 60.4 | 74.3 |
| *Trichostrongylus* | 97.0 | 93.3 | 98.4 |
| *Nematodirus* | 98.8 | 91.1 | 99.7 |
| *Hookworm* | 100 | 100 | 98.8 |
| *Chabertia* | 59.1 | 79.1 | 84.2 |
| *Cooperia* | 95.1 | 97.7 | 46.1 |
| *Trichuris* | 0 | 55.2 | 46.1 |
| Overall | 85.5 | 80.3 | 90.6 |

Test 3

The compound of Example 12, its tartrate salt, and the compound of Example 9 were tested for anthelmintic activity in pigs by the following procedure.

Ten young pigs having body weights in the range 28 to 48 kg and substantial fecal egg counts (>500) were placed in individual pens where they were given a swine grower ration and water *ad libitum* throughout a three day acclimation period and four days test period. The pigs were randomly assigned to three treatment groups of two pigs each and a control group of four pigs.

Individual dosages of the test compounds were prepared at 20 mg/kg body weight. Each individual dose of the compound of Example 12 was mixed with an equal weight of corn oil and placed in a hard gelatin capsule which was administered with the use of a balling gun. The tartrate salt of the compound of Example 12, and the compound of Example 9 were formulated by mixing each individual dose with 4 ml of corn oil, six drops of Tween 80 emulsifier ("Tween" is a trade mark) and 5 ml of water, with ultrasonic oscillation to form an emulsion. The individual dosages were administered through a stomach tube. The control pigs received plain water.

The faeces passed by each pig were collected thrice daily for four days. Twenty four hours prior to necropsy the pigs were taken off food. Four days after treatment the pigs were killed and a necropsy was conducted for the collection of all worm parasites remaining in the gastrointestinal tracts. The intestinal contents and fecal collections were washed over 0.42 mm screens. Each of the washings was stored in a 10% formalin solution. Each necropsy or fecal washing was inspected for recovery, identification and counting of all worm parasites passed following treatment or remaining at necropsy. Results are given in Table III.

TABLE III

| Parasite species | Percent Reduction | | |
|---|---|---|---|
| | Compounds of Example 12 (20 mg/kg) | Tartrate salt of compound of Example 12 (20 mg/kg) | Compound of Example 9 (20 mg/kg) |
| Roundworms | 100 | 78 | 100 |
| Whipworms | 0 | 0 | 17 |
| Nocular Worms | 90 | 24 | 94 |

Test 4

The pesticidal (insecticidal) activity of the compounds of the invention was demonstrated on aphids (*Aphis fabae*) by the following procedure.

Pairs of leaves were removed from broad bean plants and placed on filter paper inside plastic petri dishes. The leaves were sprayed on the undersurface with an aqueous formulation containing 20% by weight of acetone, 0.05% by weight of TRITON X—100 (trade mark) as wetting agent and 0.4% by weight of the compound to be tested. Varying concentrations were obtained by diluting the formulation. After spraying, the leaves were left to dry for $\frac{1}{2}$—1 hour each leaf pair was infested with ten adult aphids (*Aphis fabae*). After twenty four hours the percentages of dead and moribund aphids were recorded.

Approximate $LC_{50}$'s were obtained and are expressed as toxicity grades as follows:

| $LC_{50}$ (% active material in spray) | Toxicity grade |
|---|---|
| $> 0.6\%$ | 0 |
| $\nless 0.6$ and $> 0.2$ | 1 |
| $\nless 0.2$ and $> 0.006$ | 2 |
| $\nless 0.06$ and $> 0.02$ | 3 |
| $\nless 0.02$ and $> 0.006$ | 4 |
| $\nless 0.006$ | 5 |

Results of the tests are given in Table IV.

TABLE IV

| Compound of Example | Toxicity grade on *Aphis fabae* |
|---|---|
| 9 | 3 |
| 10 | 3 |
| 11 | 1 |
| 12 | 2 |
| 13 | 3 |
| 14 | 2 |
| 15 | 2 |
| 18 | 3 |

**Claims**

1. A dihydrothiazine derivative of formula I

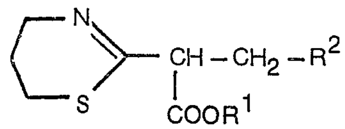

(I)

or a tautomer thereof, wherein $R^1$ is a $C_{1-10}$ alkyl, $C_{2-10}$ alkenyl, $C_{2-10}$ alkynyl, $C_{3-10}$ cycloalkyl or $C_{4-11}$ cycloalkylalkyl group optionally substituted by halogen, or is a phenyl or benzyl group optionally substituted by halogen, amino or nitro a $R^2$ is a thienyl or furyl group optionally substituted by alkyl containing from 1 to 4 carbon atoms, or a non-toxic acid-addition salt thereof, provided that when R is an alkenyl or alkynyl group the double or triple bond, respectively, is not in the $\alpha$-position to the oxygen in the group $—COOR^1$.

2. A dihydrothiazine derivative according to Claim 1 further characterised in that $R^1$ represents $C_{1-10}$ alkyl, $C_{4-11}$ cycloalkylalkyl or $C_{3-10}$ cycloalkyl.

3. A dihydrothiazine derivative according to Claim 1 or 2 further characterised in that $R^1$ represents $C_{1-6}$ alkyl or $C_{4-7}$ cycloalkylalkyl.

4. A dihydrothiazine derivative according to any one of Claims 1 to 3 further characterised in that $R^1$ represents $C_{1-4}$ alkyl or cyclopropylmethyl.

5. A dihydrothiazine derivative according to any one of Claims 1 to 4 further characterised in that $R^2$ is a 2-thienyl group optionally substituted in the 3-position by a methyl group.

6. A dihydrothiazine derivative according to any one of Claims 1 to 5 further characterised in that $R^2$ represents a 2-thienyl or 2-furyl group optionally substituted by one or more $C_{1-4}$ alkyl groups.

7. A process for preparing a dihydrothiazine derivative of formula I, as defined in any one of Claims 1 to 6, which is characterised by reacting a compound of formula II

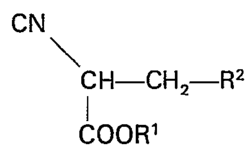

where $R^1$ and $R^2$ are as defined in Claim 1, with 3-aminopropanethiol to give a compound of formula I, optionally followed by exchanging the group $R^1$ for another group $R^1$ as defined in Claim 1.

8. An anthelmintic or pesticidal composition comprising a dihydrothiazine derivative as defined in any one of Claims 1 to 6, or when prepared by a process according to Claim 7 in association with a non-toxic carrier therefor.

9. A method of combatting pests at a locus which comprises applying to that locus a pesticidally effective amount of a dihydrothiazine derivative according to any one of Claims 1 to 6, or when prepared by a process according to Claim 7 or of a pesticidal composition according to Claim 8.

**Revendications**

1. Dérivé dihydrothiazine de formula I.

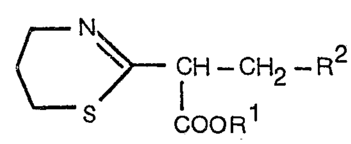

(I)

ou un de ses tautomères, dans lequel $R^1$ est un groupe alkyle en $C_1$ à $C_{10}$ alcényle en $C_2$ à $C_{10}$, alcynyle en $C_2$ à $C_{10}$; cycloalkyle en $C_3$ à $C_{10}$ ou cycloalkyle en $C_4$ à $C_{11}$, éventuellement substitué par de l'halogène, ou est un groupe phényle ou benzine, éventuellement substitué par des groupes halogènes, amino ou nitro, et $R^2$ est un groupe thiényle ou furyle, éventuellement substitué par un radical alkyle conenant de 1 à 4 atomes de carbone, ou un de ses sels non toxiques d'addition d'acide, pourvu que, lorsque $R^1$ est un groupe alcényle ou alcynyle, la double ou la triple liaison, respectivement, n'est pas dans la position $\alpha$ par rapport à l'oxygène dans le groupe $—COOR^1$.

2. Un dérivé dihydrothiazine selon la revendication 1, caractérisé en outre en ce que $R^1$ représente un groupe alkyle en $C_1$ à $C_{10}$, un groupe cycloalkylalkyle en $C_4$ à $C_{11}$ ou un groupe cycloalkyle en $C_3$ à $C_{10}$.

3. Un dérivé dihydrothiazine selon la revendication 1 ou 2, caractérisé en outre en ce que $R^1$ représente un groupe alkyle en $C_1$ à $C_6$ ou cycloalkylalkyle en $C_4$ à $C_7$.

4. Un dérivé dihydrothiazine selon l'une quelconque des revendications 1 à 3, caractérisé en outre en ce que $R^1$ représente un groupe alkyle en $C_1$ à $C_4$ ou cyclopropylméthyle.

5. Un dérivé dihydrothiazine selon l'une quelconque des revendications 1 à 4, caractérisé en outre en ce que $R^2$ est un groupe 2-thiényle éventuellement substitué en position 3 par un groupe méthyle.

6. Un dérivé dihydrothiazine selon l'une quelconque des revendications 1 à 5, caractérisé en outre en ce que $R^2$ représente un groupe 2-thiényle ou 2-furyle éventuellement substitué par un ou plus d'un groupe alkyl en $C_1$ à $C_4$.

7. Un procédé pour préparer un dérivé dihydrothiazine de formule I, tel que défini dans l'une quelconque des revendications 1 à 6, qui est caractérisé en ce que l'on fait réagir un composé de formule II

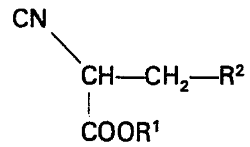

dans laquelle $R^1$ et $R^2$ sont tels que définis dans la revendication 1, avec du 3-amino-propanethiol pour fournir un composé de formule I, éventuellement suivi par un échange du groupe $R^1$ par un autre groupe $R^1$ tel que défini dans la revendication 1.

8. Une composition vermifuge ou pesticide comportant un dérivé dihydrothiazine tel que défini dans l'un quelconque des revendications 1 à 6, lorsqu'elle est préparée par un procédé selon la revendication 7 en association avec un support non-toxique.

9. Une méthode pour combattre les pestes en un lieu, qui consiste à appliquer en ce lieu une quantité efficace du point de vue pesticide d'un dérivé dihydrothiazine selon l'une quelconque des revendications 1 à 6, ou lorsqu'il est préparé par un procédé selon la revendication 7, ou d'une composition pesticide selon la revendication 8.

**Patentansprüche**

1. Dihydrothiazinderivat der Formel I

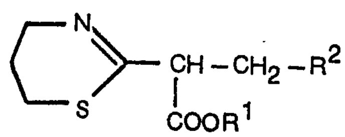

(I)

oder ein Tautomeres desselben, in der $R^1$ eine $C_{1-10}$-Alkyl-, $C_{2-10}$-Alkenyl-, $C_{2-10}$-Alkinyl-, $C_{3-10}$-Cycloalkyl- oder $C_{4-11}$-Cycloalkylalkylgruppe, die gegebenenfalls mit Halogen substituiert ist, oder eine Phenyl- oder Benzylgruppe, die gegebenenfalls mit Halogen, Amino oder Nitro substituiert ist, und $R^2$ eine Thienyl- oder Furylgruppe, die gegebenenfalls mit 1 bis 4 Kohlenstoffatome enthaltendem Alkyl substituiert ist, bedeutet, oder ein nichttoxisches Säureadditionssalz desselben, mit der Voraussetzung, daß sich die Doppel- bzw. Dreifachbindung nicht in $\alpha$-Stellung zu dem Sauerstoff in der Gruppe —$COOR^1$ befindet, wenn $R^1$ eine Alkenyl- oder Alkinylgruppe ist.

2. Dihydrothiazinderivat nach Anspruch 1, dadurch gekennzeichnet, daß $R^1$ $C_{1-10}$-Alkyl, $C_{4-11}$-Cycloalkylalkyl oder $C_{3-10}$-Cycloalkyl bedeutet.

3. Dihydrothiazinderivat nach Anspruch 1 oder 2, dadurch gekennzeichnet, daß $R^1$ $C_{1-6}$-Alkyl oder $C_{4-7}$-Cycloalkylalkyl bedeutet.

4. Dihydrothiazinderivat nach einem der Ansprüche 1—3, dadurch gekennzeichnet, daß $R^1$ $C_{1-4}$-Alkyl oder Cylcopropylmethyl bedeutet.

5. Dihydrothiazinderivat nach einem der Ansprüche 1—4, dadurch gekennzeichnet, daß $R^2$ eine 2-Thienylgruppe, die gegebenenfalls in der 3-Position mit einer Methylgruppe substituiert ist, bedeutet.

6. Dihydrothiazinderivat nach einem der Ansprüche 1—5, dadurch gekennzeichnet, daß $R^2$ eine 2-Thienyl- oder 2-Furylgruppe, die gegebenenfalls mit einer $C_{1-4}$-Alkylgruppe oder mehreren substituiert ist, bedeutet.

7. Verfahren zur Herstellung des Dihydrothiazinderivats der Formel I nach einem der Ansprüche 1—6, dadurch gekennzeichnet, daß man eine Verbindung der Formel II

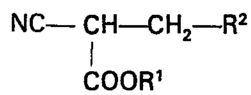

in der $R^1$ und $R^2$ wie in Anspruch 1 definiert sind, mit 3-Aminopropanthiol zu der Verbindung der Formel I umsetzt, gegebenenfalls gefolgt durch einen Austausch der Gruppe $R^1$ durch eine andere Gruppe $R^1$ nach Anspruch 1.

8. Anthelmintische oder pestizide Zusammensetzung, enthaltend ein Dihydrothiazinderivat nach einem der Ansprüche 1—6 oder hergestellt mittels eines Verfahrens nach Anspruch 7 sowie einen nichttoxischen Träger hierfür.

9. Verfahren zum Bekämpfen von Schädlingen an einem Ort, dadurch gekennzeichnet, daß man an dem Ort eine pestizide, wirksame Menge eines Dihydrothiazinderivats nach einem der Ansprüche 1—6 oder hergestellt nach einem Verfahren nach Anspruch 7 oder eine pestizide Zusammensetzung nach Anspruch 8 anwendet.